# EUROPEAN PATENT APPLICATION

(11) **EP 4 575 459 A1**
(43) Date of publication of application: **25.06.2025**
(21) Application number: 24220899.9
(22) Date of filing: 18.12.2024
(51) Int. Cl.: G01N 15/1433, G01N 15/14

(54) **A DEVICE AND A METHOD FOR ANALYZING BIOLOGICAL CELLS**

(30) Priority: 22.12.2023 EP 23219766
(71) Applicant: Imec VZW, 3001 Leuven (BE)
(72) Inventor: STAKENBORG, Tim, 3001 Heverlee (BE); VAN ROY, Willem, 3360 Bierbeek (BE); JAYAPALA, Murali, 3300 Kumtich (BE); ROTH, Sophie, 1030 Schaerbeek (BE); LUO, Zhenxiang, 3000 Leuven (BE); CHACON ORELLANA, Laura, 3210 Lubbeek (BE); ROEBROEK, Thijs, 3010 Kessel-Lo (BE)
(74) Representative: AWA Sweden AB

(57) **Abstract**

According to an aspect of the present inventive concept there is provided a device for analyzing biological cells comprising: a flow channel passing a flow of liquid carrying the biological cells to be analyzed, the flow channel comprises at least one zone associated with a cell category; at least one detector to detect information representing the biological cells passing the at least one zone; a processor configured to receive the information and to process the information by: identifying candidate biological cells; identifying tracks from the candidate biological cells; identifying candidate modified movements as events in each track; determining event related information associated with the event, the event related information comprises at least a sensed property of the candidate biological cells and/or a property defining a relation between events of at least two tracks from candidate biological cells; identifying candidate non-specific modified movements among the candidate modified movements; and removing the candidate non-specific modified movements from the candidate modified movements for each track.

## Description

### Technical field

The present description relates to a device and a method for analysis of biological cells.

### Background

Label free analysis of biological cells is important, especially in case the cells are used for post-labeling steps. The cells may be analyzed at one time-point or during several time-points. According to known methods, image analysis is used which identifies and optionally over time tracks individual cells to give information on the cells. However, the methods often require time consuming manual corrections of the tracking, to get correct data of the cells. Moreover, the tracked cells may interact with other cells or with the cell analysis chamber such that events happen which are not relevant for the analysis, or which causes events in the tracking wrongly interpreted as an event of interest. Thus, there is a need for improvements of the field.

### Summary

An objective of the present description is to enable analysis of biological cells. It is a further object of the present description to enable advanced analysis of biological cells, having high reliability. These and other objectives of the invention are at least partly met by the invention as defined in the independent claims. Preferred embodiments are set out in the dependent claims. In the following the term cell refers to a biological cell, unless otherwise stated.

According to a first aspect, there is provided a device for analyzing biological cells comprising:
a flow channel configured to pass a flow of liquid, wherein:
   the flow of liquid carries the biological cells to be analyzed,
   the flow channel comprises at least one zone,
   each zone of the flow channel is associated with a cell category, and
   each zone of the flow channel comprises at least one surface coated with molecules having an affinity and specificity to the cell category associated with the zone, the at least one surface of the zone being configured to modify, by the molecules coating the at least one surface, a movement of a biological cell belonging to an associated biological cell category of the zone as the cell, carried by the flow of liquid, passes the zone, whereby the at least one surface forms at least one cell movement modifying (CMM) surface, and whereby the modified movement of the biological cell caused by the CMM surface forms a specific modified movement;
at least one detector configured to detect information representing the biological cells, carried by the flow of liquid, passing the at least one zone;
a processor configured to receive the information detected by the at least one detector, and to process the information, wherein processing of the information comprises:
   identifying candidate biological cells;
   identifying tracks from each of the candidate biological cells;
   identifying candidate modified movements as events in each track of the candidate biological cells;
      for each event, determining event related information associated with the event, wherein the event related information comprises at least one of a sensed property of the candidate biological cells and/or a property defining a relation between events of at least two tracks from candidate biological cells;
      identifying candidate non-specific modified movements among the candidate modified movements for each track based on the event related information indicating that the event is not necessarily caused by the CMM surface; and
      removing the candidate non-specific modified movements from the candidate modified movements for each track, whereby remaining candidate modified movements for each track has an increased portion of specific modified movements.

The device enables analysis of biological cells. In particular, the device enables analysis of label-free biological cells passing the at least one zone. The analysis of the biological cells may be combined with other processes of the biological cells, such as cell sorting of the biological cells.

The device is configured to cause movement of biological cells to be affected by the CMM surface. Thus, a track of the biological cell is affected by modified movements caused by the CMM surface, such that the track may hold information of cell category of the biological cells.

However, the track of the biological cells may be affected not only by specific modified movements caused by the CMM surface. Rather, a path followed by a biological cell may be affected in other ways that may generate a risk that a track is incorrectly determined to correspond to a biological cell of interest.

When analyzing biological cells, it is of importance that the information which is basis for the analysis is reliable. Since the biological cells are in motion over the zone and since there are several biological cells being detected simultaneously and continuously, several potential errors may appear. Those errors may affect the outcome of the analysis and it is of importance for the analysis that the errors are disregarded before or during the analysis. It is a realization of the inventors that the device according to the first aspect can analyze biological cells with a higher accuracy thanks to the removal of incorrect events from the tracking of the biological cells.

Cells may be characterized by the molecules they comprise, e.g. the molecules comprised in the cell membrane. Molecules comprised in the cell membrane may e.g. be antigens. A way of checking whether a cell comprises a certain cell membrane molecule may be to let the cell react with a molecule that binds specifically to said cell membrane molecule. For example, an antigen may correspond to one or more antibodies, where the antigen and antibody bind specifically to each other. Consequently, cells may be assigned into cell categories depending on which cell membrane molecules they comprise or which molecules the cells bind to. For example, the antigen CD4 (cluster of differentiation 4) molecule in cell membranes is a glycoprotein which binds to anti-CD4 antibodies. Cells that bind to anti-CD4 antibodies (CD4 positive cells) may form one cell category. Such cells may be detected based on their modified movements when they travel through a zone with a CMM surface coated by anti-CD4 antibodies. Analogously, cells that bind to anti-CD8 (cluster of differentiation 8) antibodies (CD8 positive cells) may form another cell category. Such cells may be detected based on their modified movements when they travel through a zone with a CMM surface coated by anti-CD8 antibodies. Thus, a cell category may be a category of cells which binds to a specific molecule such as an antibody.

A biological cell may be assigned to more than one cell category. In other words, the cell membrane of a cell may have antigens against several molecules. A cell may for instance be both CD4 positive and CD8 positive. There are several antigens on a cell membrane and a combination of antibodies, or other biomarkers, against those antigens may be used to categorize cells within a larger group of cells. As an example, in a cell line the cells comprised in the cell line may belong to one, two, three, four or even more cell categories. Cell categories may also overlap, such that some of the cells belonging to a first cell category may have another marker belonging to a second cell category. The second cell category may comprise of cells belonging to the first category and other cells belonging to a third cell category.

A cell category may be a category of cells that may have differences in the expression level of a certain molecule comprised in the cell membrane. Different cells may have different levels of expression of a molecule on the cell membrane, this may be relevant to be able to categorize the cell. Even further, a cell may have different molecules expressed on the cell membrane. A combination of molecules expressed on the cell membrane and the number of molecules expressed on the cell membrane may place the cell in several cell categories, which may render a certain categorization. Each cell may belong to different combinations of cell categories.

The device may comprise more than one zone, wherein each zone may be coated with molecules having an affinity and a specificity to a respective cell category. Then, biological cells passing the multiple zones may interact with none, one or several of the CMM surfaces depending on the biomarkers on the surface of the biological cell.

For a proper identification and further analysis of the CD4 positive cells, it is of importance that each track comprises correct information. For instance, a CD4 positive cell may get stuck at a position in the zone due to a defect in the zone. Then, the tracking of that cell may not give correct information and an event corresponding to the cell being stuck in the zone should be disregarded. Another example is if a CD4 positive cell changes shape, orientation, or size for an unknown reason during the passage through the zone. Then, the event related information of the size change or shape change may indicate that the event need to be disregarded. An even further example is if a CD4 positive cell and a CD4 negative cell passes the zone simultaneously. Only the CD4 positive cell should be affected by the CMM surface of the zone, however, since the cells are very close to each other the processor may form a track wherein the tracked cell changes from the CD4 positive cell to the CD4 negative cell within the track. This would give information from both the CD4 positive cell and the CD4 negative cell in one track. Such incorrect information should be disregarded. Even more examples of tracking errors will be discussed throughout the application.

The molecules coating at least one CMM surface of the at least one zone of the flow channel may comprise at least one of: antibodies, aptamers, antibody fragments, peptides, molecularly imprinted polymers, receptors or lectins. Antibodies, aptamers, antibody fragments, peptides, molecularly imprinted polymers, receptors or lectins may have an affinity to specific cell categories but not to other cell categories. Antibodies, aptamers, antibody fragments, peptides, molecularly imprinted polymers, receptors or lectins may bind to specific cell categories but not to other cell categories. Antibodies, aptamers, antibody fragments, peptides, molecularly imprinted polymers, receptors or lectins may have a high sensitivity in binding to a cell category. Thus, the percentage of true positive binding events may be high. Antibodies, aptamers, antibody fragments, peptides, molecularly imprinted polymers, receptors or lectins may have a high specificity in binding to a cell category. Thus, the percentage of false positive binding events may be low. Alternatively, the molecules coating at least one CMM surface of the at least one zone of the flow channel may comprise other molecules than antibodies, aptamers, antibody fragments, peptides, molecularly imprinted polymers, receptors and lectins, e.g. comprise other molecules that binds specifically to a cell membrane molecule.

A CMM surface may be configured to modify the movement of the cell belonging to the associated cell category of the zone by modifying e.g. the speed, direction or path of cells comprised in the cell category. For example, the at least one CMM surface of at least one zone of the flow channel may be configured to modify the movement of the cell belonging to the associated cell category of the zone by slowing down the speed of the cell movement. This may be made by the molecules binding and releasing to the cell belonging to the cell category such that the speed slows down. As a second example, the at least one CMM surface of at least one zone of the flow channel may be configured to modify the movement of the cell belonging to the associated cell category of the zone by binding and releasing the cell belonging to the associated cell category of the zone. Such a movement may be termed a bind-release movement. Thus, the CMM surface may be configured not to bind a cell indefinitely. Binding and releasing the cell may temporarily halt the cell along its travel. Such a modified motion in the form of a modified speed may be detectable by the at least one detector. The cell may not necessarily bind and release at the same point of the CMM surface. The CMM surface may be configured to let a cell bind to the CMM surface, roll a distance along the CMM surface, and then release from the CMM surface in a bind-roll-release movement. The cell may e.g. roll along a surface in a direction different from the direction of the flow of liquid. Thus, the direction or path of a cell belonging to a category associated with the CMM surface may be different from the path of a cell belonging to a category not associated with the CMM surface. Such a modified motion in the form of a modified direction or path may be detectable by the at least one detector.

In some embodiments the flow channel may comprise more than one zone. The at least one zone of the flow channel may comprise at least a first zone and a second zone, wherein a composition of the molecules coating the at least one CMM surface of the first zone differs from a composition of the molecules coating the at least one CMM surface of the second zone. Within the flow channel the second zone may be arranged downstream from the first zone.

Several zones with different CMM surfaces may enable modified movements of the cells belonging to multiple cell categories. The combination of information regarding the modified movements of cells in different zones may also enable complex analysis of the cells. For instance, a biological cell may be subject to modified movements in several zones and different biological cells may be distinguished from each other based on in which combination of zones the biological cells are subject to modified movements.

The information on the biological cells passing through the zone and thereby having a modified movement is detected by the detector and the information is further received by the processor. The processor is processing the information to analyze the modified movement. For this analysis to be accurate, removing of potential non-specific modified movements from the tracks such that the non-specific modified movements are not included in the analysis may greatly improve accuracy of the analysis.

The at least one detector is configured to detect information representing the biological cells passing the at least one zone.

The at least one detector may be configured to acquire images of the biological cells in the flow channel. The at least one detector may be configured to acquire an image series representing a time-sequence of images of the biological cell. From the images, the processor may process information regarding the biological cells.

The image acquired by the detector may be a 2D array of intensities, such as a hologram. The hologram may then include information of the area detected by the detector. The hologram may be used to reconstruct an image for the user to see. However, the processor may use the information in the hologram without reconstructing an image,

It should be understood that the detector may comprise multiple image sensors. The multiple image sensors may image a larger area than a single image sensor could. Each image sensor may have its own light source. Alternatively, two or more image sensors may share a light source. The field of view of an image sensor may or may not overlap with the field of view of another image sensor.

The processor is configured to receive the information detected by the at least one detector. Thus, the processor may be configured to analyze a time-sequence of information of the biological cells and detect the cell as exhibiting the at least one modified movement based on the time-sequence of information.

The processor may be configured to detect one modified movement of a cell in a zone, e.g. one bind and release event, a halt of motion, a change of speed, a change of direction, or a change of path, from the information received from the detector, and thereby identify the cell as belonging to the cell category associated with the zone. Alternatively, the processor may be configured to process the information from the at least one detector and identify a threshold number of modified movements of a cell in a zone, e.g. the cell displaying at least 2, at least 5, or at least 10 bind and release events within the zone, and thereby identify the cell as belonging to the cell category associated with the zone. The processor may be configured to identify modified movements of two or more cells simultaneously, as they move across a CMM surface of the flow channel.

The candidate biological cells are identified from the information detected by the at least one detector. The candidate biological cells may be biological cells belonging to the cell category of the zone. The candidate biological cells may be a biological cell not belonging to the cell category of the zone. The candidate biological cell may however also be non-cell. The non-cell could be a debris in the flow of liquid, a dead or injured cell, an artefact in the CMM surface of the zone or an imaging artefact interpreted by the detector as a cell.

The processor of the device is configured such that it processes information acquired by the detector such that it may decide the probability that a candidate biological cell is actually a biological cell or not. It may further process the information such that it may decide the probability if it is a biological cell of interest or a biological cell not of interest. Returning to the example of CD4 positive cells and CD4 negative cells, the analysis may for instance only be interested in CD4 positive cells. Then, the CD4 negative cells may be removed from the analysis as bological cells not of interest. The CD4 negative cells may be identified based on its behavior while passing the zone. For instance, the CD4 negative cells may have a more undisrupted and faster passage through the zone than the CD4 positive cell.

Each candidate biological cell may be represented by a track that defines a path of a movement of the candidate biological cell through the at least one zone of the flow channel. The track may represent the position of a detected candidate biological cell in the flow channel over a time-sequence during which the candidate biological cell passes the zone. Thus, the movements of the each of the candidate biological cell passing the zone is tracked as well as the time the candidate biological cell spends in the zone.

The track may represent a set of data points, where each data point represents a timepoint and the position of the candidate biological cell at least in two dimensions but even in three dimensions. Thus, each track comprises information of x, y, and optionally z, and t, where x, y and z are spatial coordinates and t are the timepoint.

The detector acquires information on movement in space and time of the candidate biological cell and may provide such information to the processor. The processor may further acquire information of other properties of the candidate biological cells. The information may for instance be size, shape and solidity. The information may give the processor, through the detector, enough information to decide the probability that a candidate biological cell is a biological cell of interest, a biological cell not of interest or a non-cell.

In addition to the time dependent spatial information about the candidate biological cell, information about the morphological properties such as morphology of the candidate biological cell for each time may be acquired and processed by the processor. The information about the morphological properties such as morphology of the candidate biological cell may be acquired for one time point or several time points, but it need not necessarily be acquired for all time points.

For each of the tracks of the candidate biological cells, morphology events may be identified. The morphology event may be a size, shape, orientation, granularity, homogeneity, smoothness, patchiness, transparency, scattering, brightness or solidity of the candidate biological cells. The morphology event may as well be a change in size, shape, orientation, granularity, homogeneity, smoothness, patchiness, transparency, scattering, brightness or solidity of the candidate biological cells. The morphology event may for instance indicate clustering of cells, i.e. that several cells are stuck together for a shorter or longer time. The morphology event may as well indicate that a cell is stuck to debris in the flow channel. Altogether, the morphology event may indicate that something has affected the candidate biological cells in a way such that the tracking is not correct. Thus, if it can be determined that the morphology event is not necessarily caused by the CMM surface, part of a track, including the morphology event, or a full track which includes the morphology event not necessarily caused by the CMM surface can be removed.

For each of the track of the candidate biological cells, candidate modified movements are identified as events.

A candidate modified movement may be a movement induced by a biological cell belonging to the associated biological cell category of the zone and thereby the candidate modified movement is a specific modified movement caused by the cell passing the CMM surface. In this case, the candidate biological cell is a biological cell matching the cell category of the specific zone and the candidate modified movement is a modified movement caused by the CMM surface. A candidate modified movement may however also be an event in the track that may be mistaken for a modified movement caused by the CMM surface but which is in fact not caused by the CMM surface. It may for instance be a candidate biological cell not belonging to the associated cell category, which moves over the zone making random movements. It may further be a candidate biological cell being a non-cell, such as a debris in the flow of liquid. The non-cell may move over the zone, without being affected by the CMM surface. All of those movements will be tracked, and the information will be analyzed, together with the tracks from the biological cells belonging to the associated biological cell category of the zone, if it is not removed.

According to an embodiment, any movement through the at least one zone may be identified as a candidate modified movement.

The candidate modified movements are identified as events in each of the tracks. A track may comprise very few, such as 0 or 1 event. This may occur if the cell passes the zone with an even speed and without any interaction. Each track may comprise several events. It may for instance comprise 2, 10 or 50 events. Each event may represent different candidate modified movements of the candidate biological cells. For instance, a candidate biological cell may change its movement several times while passing the zone. This may render several events of candidate modified movements.

For each of the events, event related information associated with the event is determined. The event related information comprises at least one of a sensed property of the candidate biological cells and/or a property defining a relation between events of at least two tracks from candidate biological cells. By event related information, it should be understood that it may be an actual property of the candidate biological cell or a property defining the relation between events of at least two tracks from two candidate biological cells or it may be a change in the sensed property of the candidate biological cell or a change in the property defining the relation between events of at least two tracks from two candidate biological cells.

The event related information is used to determine what events that are to be kept and what events that are to be removed.

Among the candidate modified movements for each track, candidate non-specific modified movements are identified. The identification is made based on the event related information where the event related information indicates that the event is not necessarily caused by the CMM surface. In other words, candidate modified movements not necessarily caused by the CMM surface are identified as non-specific modified movements. Candidate modified movements caused by the CMM surface, but where the track further comprises events not caused by the CMM surface, may also be identified as non-specific modified movements.

According to an embodiment, all events identified in a track before and/or after an event identified as a candidate non-specific modified movement may also be identified as candidate non-specific modified movements.

The candidate non-specific modified movements are removed from the candidate modified movements for each track. Thereby, the remaining candidate modified movements for each track have an increased portion of specific modified movements, that is modified movement that actually correspond to the movement of the biological cell being modified by the CMM surface. Thus, each track may comprise several events originating from specific modified movements but also events originating from non-specific modified movements. The events from the non-specific modified movements may be removed. By this, the device allows for analysis of biological cells where the remaining modified movements in the tracks correspond only or at least to a high degree to movements of the biological cells being modified by the CMM surface. Thus, events registered as not being from biological cells having a modified movement caused by the CMM surface are removed. By this, the proportion of specific modified movements among the events in the tracks increases. This implies that a track representing movement of a biological cell that does not belong to the cell category associated with the at least one zone or a track representing movement of non-cells may have no or very few remaining events and may therefore allow correct determination of the tracks as not belonging to a biological cell of interest. Hence, the device allows information of interest, that is information corresponding to specific modified movements, to be distinguished from non-specific modified movements and/or irrelevant tracks or parts of tracks. This implies that the removing of the candidate non-specific modified movements from events in the tracks and/or the removing of tracks or parts of tracks based on morphology events may be viewed as removing noise from the tracks.

Removing of a candidate non-specific modified movement may correspond to a part of a track being removed from consideration. For instance, if two tracks meet such that there is an event associated with an encounter of two cells, it may not be possible to determine which parts before and after the event that correspond to the same cell. In such case, an entire part of the track before or after the event may be removed, wherein the removing also removes the non-specific modified movement corresponding to the encountering of the cells. As an alternative, the removing of the candidate non-specific modified movement may simply correspond to removing the event from consideration.

It should be realized that the term "candidate biological cell" is used herein as representing an object that is represented by the track. The information from the detector may comprise information not only relating to biological cells of interest in the flow of liquid. Rather, the object that is tracked may be a biological cell that does not belong to the associated cell category of the at least one zone or the object may be a non-cell. Based on the removing of non-specific modified movements, determining which tracks that truly correspond to biological cells that belong to the associated cell category of the at least one zone is facilitated.

According to an embodiment, the processing of the information may further comprise: analyzing each of the remaining part of the tracks based on remaining candidate modified movements of the track to determine whether the track represents movements of the biological cell belonging to the associated biological cell category of the zone.

Thus, the analysis allows identifying the tracks that truly represent biological cells belonging to the associated biological cell category of the zone.

Analysis of the remaining part of the tracks may correspond to determining a number of events remaining in the tracks, such as comparing the number of events to a threshold. For instance, if the number of events is larger or equal to the threshold, the track may be determined as representing movements of the biological cell belonging to the associated biological cell category of the zone.

However, it should be realized that the processor may not necessarily perform such analysis. The processor may be configured to improve a signal-to-noise ratio relating to information of the tracks based on removing candidate non-specific modified movements. The information of the tracks may be transmitted to an external unit for further processing and analysis therein. As yet another alternative, information of the tracks after the non-specific modified movements have been removed may be displayed to a user for manual analysis of the tracks. The manual analysis may then be greatly augmented by the signal-to-noise ratio of the tracks being improved.

The processing of the information may give options for a further processing.

As a first example, the processing may result in a decision to analyze this cell again, thus send it again though the device, or decide to measure it in a different way.

As a second example, the processing may result in a decision to remove the track from the analysis. This may for instance be used in a QC (quality control) application to determine which fraction of cells is positive for the at least one zone. This may for instance be a ratio between the number of cells associated to the cell category divided total number of candidate biological cells.

As a third example, if the device is further complemented with a cell sorter or cell selector arranged after the analysis zone, this may require a high yield of cells associated with the cell category of the zone.

As a fourth example, if a further use case requires a high purity, i.e. the collected biological cells all need to be associated with the cell category of the zone, cells consider it as not being associated to the cell category may be rejected.

According to one embodiment the at least one detector may be an optical sensor or the device may further comprise an optical sensor. The processor may be configured to process information detected by the optical sensor for determining an optical property of each of the candidate biological cells. The property of the candidate biological cells may comprise an optical property of the candidate biological cell. The optical property of the candidate biological cell may be event related information used to identify candidate non-specific modified movements.

The optical sensor may be a CMOS image sensor.

The information detected by the optical sensor may be a one-time measurement of the optical property or the information may be a time-sequence of information such that a change in the optical property may be determined.

The processor may be configured to identify tracks in the information detected by the at least one detector. This may be performed based on optical detection of information, such that the optical sensor may be used both for detecting the information to allow tracks to be determined as well as for detecting optical properties of the candidate biological cells. However, it should be realized that information relating to tracks may be acquired by detecting changes in electrical properties in the flow channel associated with movements of objects therethrough. In such case, the optical sensor may be used for detecting additional information of the candidate biological cell.

The optical property may provide information of the candidate biological cell. For instance, morphology of the candidate biological cell may be determined, which may be used as information allowing determination whether the track represents the biological cell of interest.

It should further be realized that the sensed optical property of the biological cell may change. For instance, the biological cell may be thin and difficult to distinguish in the time-sequence of images such that the biological cell may be temporarily not visible in the time-sequence. This implies that the sensed optical property may correspond to determining a, disappearing, a temporarily disappearing or fading of the biological cell from the time-sequence allowing a non-specific modified movement based on such disappearing or fading to be identified.

According to one embodiment the optical property of the candidate biological cells may comprise the size, shape, orientation, granularity, homogeneity, smoothness, patchiness, transparency, scattering, brightness or solidity of the candidate biological cells.

The optical property may be used to determine the event related information associated with each event of each track. The optical property may be used to determine if the event is likely caused by an interaction between the candidate biological cell and the CMM surface, or if the event is likely caused by any other non-specific modified movement.

The optical property may for instance be used to determine relations between at least two cells.

According to one embodiment the at least one detector may be an electrical sensor or the device may further comprise an electrical sensor. The processor may be configured to process information detected by the electrical sensor for determining an electrical property of each of the candidate biological cells. The property of the candidate biological cells may comprise an electrical property of the candidate biological cell.

The electrical sensor may be an electrical impedance sensor. The electrical sensor may register electrical signals as a response from the candidate biological cells after an external electrical stimulation is applied.

The electrical property may be used to determine the event related information associated with each event of each track. The electrical property may be used to determine if the event is caused by an interaction between the candidate biological cell and the CMM surface, or if the event is caused by any other non-specific modified movement. The electrical property may for instance be used to determine a change in size, shape or morphology of the candidate biological cell. A change in size, shape, morphology may cause a modified movement, and therefore the identification of the change in size, shape, morphology before the modified movement may help in determining that the modified movement is not caused by interaction with the CMM surface.

The electrical property may for instance be used to determine relations between at least two cells.

According to one embodiment the electrical property of the candidate biological cells may comprise the electrical impedance, at one or more frequencies of an applied AC field, of the candidate biological cells, wherein the electrical impedance of the candidate biological cells is used to determine e.g. the size, membrane capacitance or cytoplasm conductivity of the candidate biological cells.

For instance, an AC electric field may be applied, e.g. between a 2D array of electrodes at the bottom of the zone and a counter electrode at the top of the flow channel. This may allow for measurement of the impedance of the liquid as well as any candidate biological cells being between each electrode and the counter-electrode.

According to one embodiment the candidate biological cell may be identified as a biological cell of interest, a biological cell not of interest or a non-cell, based on the event related information comprising the property of the candidate biological cells.

Thus, the candidate biological cell may be a biological cell belonging to an associated biological cell category of the zone. The candidate biological cell may be a biological cell not belonging to an associated biological cell category of the zone. The candidate biological cell may be a biological cell having a different shape, e.g. a non-spherical cell, indicating that the cell for instance may be injured and thereby not of interest for the analysis. Further, the candidate biological cell may be a debris in the flow of liquid or an imaging artefact, and thereby be identified as a non-cell.

According to one embodiment, determining event related information may comprise determining a spatial and/or temporal property of the event, and determining a relation between events of at least two tracks from candidate biological cells may comprise comparing spatial and/or temporal properties of events of the at least two tracks.

In other words, the event related information may have information on where or when the event happened. For instance, the event related information can reveal if two or more candidate biological cells have been at the same position at the same time or if two or more candidate biological cells were very close to each other at the same time.

Moreover, the event related information can reveal if an event happens at the same position but at different times for several candidate biological cells or if an event happens at the same time but at different positions for several candidate biological cells.

For example, an event happening at the same time for several candidate biological cells may be due to a sudden flow instability. For example, an event happing for several candidate biological cells at the same location may be the result of inaccuracies in the flow cell.

Moreover, the information may be used to determine if there are large and/or fast changes in the position of the candidate biological cells. This may give a large change in spatial position and thus be comprised in a spatial property of the event.

Even further, the information may be used to identify sudden changes in orientation. This may be comprised in the spatial property of the event.

This information may be used to determine events that are not necessarily caused by the CMM surface. The information may then be used to reduce the number of events which otherwise falsely would have been characterized as a specific modified movement.

According to one embodiment, determining event related information may comprise determining a property based on the candidate biological cells for at least two tracks being within a spatial distance to each other smaller than a threshold.

Thus, the event related information may indicate that at least two candidate biological cells have been close to each other. This may indicate that the candidate biological cells have interacted with each other. The interaction between the at least two cells may wrongfully be interpreted as a specific modified movement caused by the CMM surface, leading to an error of the tracking. Thus, the determination of the interaction between the at least two candidate biological cells may reduce the number of false events based on interactions.

The threshold may be measured as a center-to-center distance, thus as the distance between the center point of each of the candidate biological cells. The threshold may then be 1x or 1.5x or 2x or 5x of the diameter of the candidate biological cells being close to each other. The diameter of the candidate biological cells may be 8-10 µm. The threshold may on the other hand be 10 or 15 or 20 or 50 µm.

The threshold may be measured as a edge-to-edge distance, thus as the distance between the edge of each of the candidate biological cells. The threshold may then be 0.1x or 0.5x or 1x or 5x of the diameter of the candidate biological cells being close to each other. The diameter of the candidate biological cells may be 8-10 µm. The threshold may on the other hand be 1 or 5 or 10 or 20 µm.

According to one embodiment the property defining a relation between events of at least two tracks from candidate biological cells may indicate encountering of at least two candidate biological cells.

The encountering could be between two biological cells, between one biological cell and debris or an imaging artefact, or between two parts of debris. All of those encounters may otherwise be identified as a specific modified movement.

The encountering may include two candidate biological cells interacting with each other, or moving above and below each other.

It should be understood that removal of the candidate non-specific modified movements identified from the property defining a relation between events of at least two tracks may lead to removal of candidate non-specific modified movements from one of the tracks or from several tracks in the relation. Thus, the removal may be from one, two, three or more tracks. Further, it should be understood that not all tracks involved in an encounter may have parts removed. Thus, it may be such that all information form one track may be left without a removal, while another track has parts removed.

According to one embodiment the property defining a relation between events may indicate that an association between a particular track and a particular candidate biological cell is lost or generated inside the zone.

When at least two candidate biological cells are close to each other, or when tracks of the two candidate biological cells cross each other, the track representing a candidate biological cell before the event may move to another candidate biological cell after the event. The track may for instance in two images following each other move from a first candidate biological cell to a second candidate biological cell based on that the second candidate biological cell in the second image is at a location close to where the first candidate biological cell was in the first image. This would of course render all of the previous or following events of the two tracks being wrong, since each of the tracks now represent another candidate biological cell than in the first place.

It should be understood that the tracking of one candidate biological cell may accidentally be moved to another candidate biological cell which have not yet been assigned with a tracking. This will give rise to the same problems as identified above, i.e., that one tracking may comprise information of two (or more) candidate biological cells.

A tracking may, in the same way, move from a candidate biological cell to a debris. Again, this would make the tracking comprise information from more than the candidate biological cell identified at the start of the tracking. According to one embodiment, the processor may additionally identify the length of each track. Thus, the actual length a candidate biological cell has moved when being tracked. This may be used to remove tracks comprising errors. For instance, a very short length of a track may indicate that a tracking was interrupted during the passage of the candidate biological cell through the at least one zone. This may occur due to a cell is fainting in the images during the passage of the at least one zone.

According to one embodiment, the processor may further gather information on the start and/or end of the tracking. If this appears to be away from the entrance and/or exit of the at least one zone, it may further indicate that a candidate biological cell has lost its tracking while passing through the at least one cell and the information of the tracking may comprise errors leading to it being disregarded.

According to one embodiment, determining event related information may comprise determining whether a multitude, such as at least four, of events in respective tracks share a spatial and/or a temporal property.

There may for instance be a clustering of events in space. Thus, the tracks of a plurality of candidate biological cells have events at the same location of the zone. This may indicate that there is an invisible defect, debris or stuck cell at the specific location. Thus, events happening at that location would not be specific modified movements caused by the CMM surface and may be removed from each of the tracks.

Moreover, a specific time-point in the track may be removed if the event related information determines with a high probability that the event was not caused by the CMM surface.

There may further for instance be a clustering of events in time. Thus, a plurality of events shows up at the same time in tracks of a plurality of candidate biological cells. This may for instance happen due to an irregular flow of the flow of liquid. Thus, events happening on the same time may not be specific modified movements caused by the CMM surface and may be removed from each of the tracks.

The multitude of events may be 2, 10, 100 or 1000 events depending on the analysis for the device.

There may be a clustering of events both in time and space, such that several candidate biological cells may cluster together at a certain place and at a certain time. This may not be specific modified movements caused by the CMM surface and may be removed from each of the tracks. Instead, parts of or the entire track may be removed to remove the information not caused by the CMM surface,

According to one embodiment the processor may be configured to determine that the entirety of a track before and/or after an event is to be disregarded based on the determined event related information of the event of the track.

For instance, event related information may provide general information relating to the candidate biological cell. For instance, the sensed property of the candidate biological cell may indicate that the candidate biological cell is not a biological cell of interest. As an example, this may be determined based on size or morphology of the candidate biological cell which may be determined based on a sensed property. In such case, the processor may not need to further process the track and may instead completely disregard the track.

As another alternative, the event related information may be such that it is realized that the entire track comprises so many errors that it is of no use. It may further be such that it is realized or that it may not be ascertained whether the track changes from representing a first candidate biological cell to a second candidate biological cell. The entire track may then be disregarded since it may comprise information of two different candidate biological cells.

Moreover, if a candidate biological cell is stuck in the zone the entire track may be disregarded since it may not have proper data on how the cell behaves when passing the zone.

Even further, if the event related information reveals that there may be a switch of tracks between two or more candidate biological cells, the entire tracks may be removed as each of the tracks do not longer comprise information from one cell, but from at least two cells. This may alternatively remove the part of the track after the event where the switch of tracks are identified, as the tracking before the switch may comprise correct and relevant information of the candidate biological cell.

It should be understood that there are more events that may be disregarded as a full track and the above should only be seen as examples.

According to one embodiment the device further comprises:
a light source configured to illuminate a biological cell in the flow channel such that an interference pattern is formed by interference between light being scattered by the illuminated biological cell and non-scattered light from the light source; and
wherein the detector comprises an image sensor configured to detect an image series that represents a time-sequence of interference patterns of the illuminated biological cell.

Such a detector may enable a high cell throughput. A detector operating according to the principles of digital holographic imaging may have a large field of view since a lens may not be needed between the image sensor and the flow channel. Further, a detector operating according to the principles of digital holographic imaging may have a large depth of focus. Consequently, the detector may monitor a large area or a large volume, of the flow channel, for cells exhibiting modified movements. Monitoring a large area or volume simultaneously makes it possible to maintain a high flow rate and a high cell throughput.

The non-scattered light from the light source may be passed along a common optical path with the light being scattered by the cells. Thus, the interference pattern may be formed between scattered and non-scattered light at the image sensor in a so-called in-line holography set-up. Thus, the scattered and non-scattered light may share the same light path. However, according to an alternative, the non-scattered light may be passed along a separate reference light path, which is combined with the light having been scattered by the cells before reaching the image sensor. In such case, the light scattered by the cells may be either forward or backward scattered light.

It should be realized that the interference pattern may be used for reconstructing images of the flow channel. Thus, such reconstructed images may be provided to the processor as information representing the biological cells carried by the flow of liquid. However, the processor may be able to identify the candidate biological cells and the tracks therefrom in the time-sequence of interference patterns without the interference patterns being converted to reconstructed images.

As an alternative to a detector operating according to the principles of digital holographic imaging, the detector may use imaging with a lens to detect cells exhibiting modified movements. Thus, the detector may detect an image series representing a time-sequence of a cell in the flow channel instead of a time-sequence of interference patterns of the cell.

According to a second aspect, there is provided a method for analyzing biological cells, the method comprising:
receiving information detected by at least one detector representing biological cells, carried by a flow of liquid, to be analyzed passing at least one zone in a flow channel, wherein
   each zone of the flow channel is associated with a cell category,
   each zone of the flow channel comprises at least one surface coated with molecules having an affinity to the cell category associated with the zone, the at least one surface of the zone being configured to modify, by the molecules coating the at least one surface, a movement of a biological cell belonging to the associated cell category of the zone as the biological cell carried by the flow of liquid passes the zone, whereby the at least one surface forms at least one biological cell movement modifying (CMM) surface, and whereby the modified movement of the biological cell caused by the CMM surface forms a specific modified movement,
processing the received information, wherein said processing of the information comprises:
   identifying candidate biological cells in the flow channel;
   identifying tracks from each of the candidate biological cells;
   identifying candidate modified movements as events in each track of the candidate biological cells;
      for each event, determining event related information associated with the event, wherein the event related information comprises at least one of a sensed property of the candidate biological cells and/or a property defining a relation between events of at least two tracks from candidate biological cells;
      identifying candidate non-specific modified movements among the candidate modified movements for each track based on the event related information indicating that the event is not necessarily caused by the CMM surface; and
      removing the candidate non-specific modified movements from the candidate modified movements for each track, whereby remaining candidate modified movements for each track has an increased portion of specific modified movements.

This aspect may generally present the same or corresponding advantages as the former aspect.

The method allows for analysis of biological cells with high accuracy and high throughput.

Thanks to the processing of the information received from the at least one detector to the processor, errors in the events of the tracking may be removed. The errors may comprise events where the candidate biological cell changes properties such as size, shape or solidity. The errors may comprise events where the candidate biological cell changes electrical properties.

Many events may arise at the same location of the zone, such that the information is a spatial information. Many events may on the other hand take place during the same time and the event information is time resolved.

The event information may comprise information on encountering between at least two candidate biological cells. Thus, at least two biological cells may be in close proximity to each other leading to interactions between the at least two cells which may give errors in the information. Further, the track representing a candidate biological cell may move from one candidate biological cell to another if the two candidate biological cells are very close to each other.

The method allows for removal of the events of each track which may comprise event information not being related to the modified movements induced by the CMM surface.

According to a third aspect, there is provided a computer program product comprising computer-readable instructions such that when executed on a processor, the computer program product will cause the processor to perform the method according to above.

This aspect may generally present the same or corresponding advantages as the former aspects.

The computer program product may implement the method in a processing unit, which may be a dedicated processing unit for performing the method or may be a general-purpose processing unit which may be able to perform the method based on the computer program product.

The computer program product may comprise a computer-readable medium on which the computer-readable instructions are stored. The computer program product may thus be provided as a non-transient computer program product stored on any tangible medium. Alternatively, the computer program product may be provided as a signal carrying the computer-readable instructions for allowing the computer program product to be loaded into a memory accessible to the processing unit.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1a is a device for analysis of biological cells.
Fig. 1b is a device for analysis of biological cells.
Fig. 2a is a device for analysis of biological cells.
Fig. 2b is a device for analysis of biological cells.
Fig. 3 is a device for analysis of biological cells.
Fig. 4a is a set of candidate biological cells.
Fig. 4b is a device for analysis of biological cells.
Fig. 4c is a device for analysis of biological cells.
Fig. 4d is a schematic view of events.
Fig. 4e is a device for analysis of biological cells.
Fig. 4f is a schematic view of events.
Fig. 4g is a schematic view of events.
Fig. 5 is a flow chart of a method for analyzing biological cells.

### Detailed description

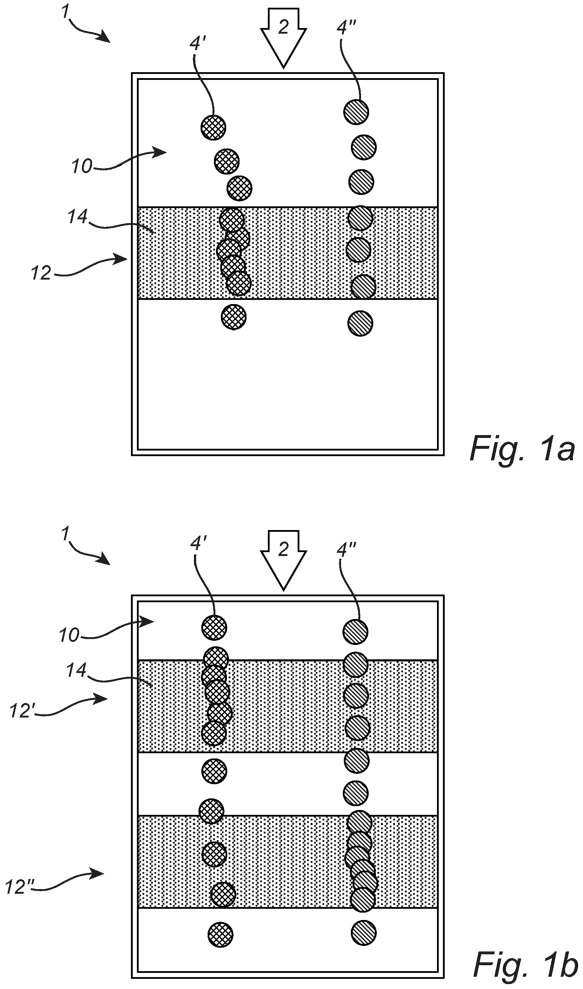

Figs 1a and 1b illustrate a flow channel 10 of a device 1 for analyzing biological cells 4. The flow channel 10 is configured to pass a flow 2 of liquid carrying the cells 4 to be analyzed. The flow channel 10 may be a microfluidics channel. The flow channel 10 may be defined by conventional lithography and sealed with a transparent cover slide, e.g. glass, PDMS, or polycarbonate. The top part of the flow channel 10 may be covered with a cover slide, e.g. a glass slide, bonded to the PDMS. The bottom part of the flow channel 10 may be at least partially transparent. It may comprise glass or quartz.

In Fig. 1a, the illustrated flow channel 10 comprises one zone 12. The flow 2 of liquid and the biological cells 4 passes the zone 12. The bottom part of the zone 12 are covered with molecules such that they form a cell movement modifying (CMM) surfaces 14. The CMM surfaces 14 comprise molecules having an affinity to a cell category.

In Fig. 1a, in the illustrated flow channel 10, the CMM surfaces 14 of the zone 12 are coated by molecules having an affinity to biological cells 4' of a first category. Thus, the zone 12 may be considered to be associated with the first category.

In Fig. 1b, in the illustrated flow channel 10, the CMM surfaces 14 of the first zone 12' are coated by molecules having an affinity to cells 4' of a first category. Thus, the first zone 12' may be considered to be associated with the first category. In the illustrated flow channel 10, the CMM surfaces 14 of the second zone 12" are coated by molecules having an affinity to cells 4" of a second category. Thus, the second zone 12" may be considered to be associated with the second category.

Except for the number of zones, the device 1 may be configured in the same way regardless of the device 1 having one or several zones 12. It should be understood that the device may have more than two zones.

The molecules of the CMM surfaces 14 may be e.g. antibodies, aptamers, antibody fragments, peptides, molecularly imprinted polymers, receptors or lectins. For example, the molecules of the CMM surface 14 of the first zone 12' may be anti-CD4 antibodies and the molecules of the CMM surface 14 of the second zone 12" may be anti-CD8 antibodies. Thus, a first cell category may be defined as biological cells 4' having a cell membrane comprising CD4 antigens ("CD4 positive cells") and a second cell category may be defined as biological cells 4" having a cell membrane comprising CD8 antigens ("CD8 positive cells").

It should be realized that CMM surfaces may be provided on side walls or on the top surface of the flow channel instead or in addition to on bottom part of the channel 10.

As illustrated in Fig. 1a and 1b, the molecules of the CMM surfaces 14 may modify the speed of the movement of the cells 4. The modified movements may be expressed as cells 4 have a reduced speed if they interact with the CMM surfaces 14 of a zone 12.

In Fig. 1a, a biological cell 4' of the first category have a slower speed in the zone 12 compared to the speed of the biological cell 4" of the second category.

In Fig. 1b, a biological cell 4' of the first category have a slower speed in the first zone 12' than in the second zone 12". Analogously, a biological cell 4" of the second category have a slower speed in the second zone 12" than in the first zone 12'.

However, one candidate biological cell 4', 4" may belong to more than one cell category. Thus, one candidate biological cell 4', 4" may belong to both the associated cell category with the first zone 12' and the second zone 12".

The molecules may bind and release a cell 4 belonging to the associated cell category of the zone 12. For example, an anti-CD4 antibody may bind to a cell 4 having a cell membrane comprising CD4 antigens, and subsequently release the cell. By this, the speed of a biological cell 4' of the first category moving through the first zone 12' may be reduced such that it is possible to identify the cell as belonging to the first category. The movements of a biological cell 4" of the second category moving through the second zone 12" may change analogously.

When a biological cell belongs to a category associated with a zone the biological cell may, in that zone, exhibit e.g. a bind and release event, a halt of motion, a change of speed, a change of direction, or a change of path.

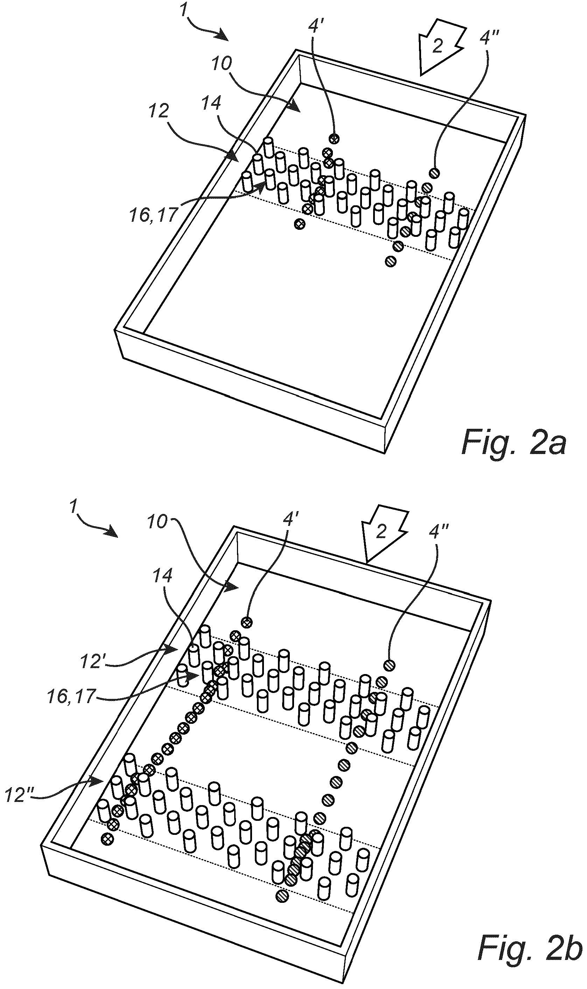

Fig. 2a and 1b illustrates a flow channel 10 of a device 1 for analyzing biological cells 4. The device 1 have many similarities to the device 1 described in Figs. 1a and 1b, thus only differences between the figures will be disclosed. Fig. 2a, the illustrated flow channel 10 comprises one zone 12. The flow 2 of liquid and the biological cells 4 passes the zone 12. The zone 12 comprises obstacles 16, in the figure obstacles 16 in the form of pillars 17.

In Fig. 2b, the illustrated flow channel 10 comprises two zones 12. The flow 2 of liquid and the biological cells 4 first passes a first zone 12' and then a second zone 12". Each zone 12 comprises obstacles 16, in the figure obstacles 16 in the form of pillars 17.

Except for the number of zones, the device 1 may be configured in the same way regardless of the device 1 having one or several zones 12. It should be understood that the device may have more than two zones.

In the illustrated device 1, the side walls of the pillars 17 form lateral surfaces that obstruct the path of the biological cells 4. Thus, the biological cells 4 repeatedly hit the side walls of the pillars 17 during their travel through the flow channel 10. Further, the side walls of the pillars 17 form cell movement modifying (CMM) surfaces 14. The CMM surfaces 14 comprise molecules having an affinity to a cell category.

In Fig. 2 a, in the illustrated flow channel 10, the CMM surfaces 14 of the zone 12 are coated by molecules having an affinity to biological cells 4' of a first category. Thus, the zone 12 may be considered to be associated with the first category.

In Fig. 2b, in the illustrated flow channel 10, the CMM surfaces 14 of the first zone 12' are coated by molecules having an affinity to cells 4' of a first category. Thus, the first zone 12' may be considered to be associated with the first category. In the illustrated flow channel 10, the CMM surfaces 14 of the second zone 12" are coated by molecules having an affinity to cells 4" of a second category. Thus, the second zone 12" may be considered to be associated with the second category.

As illustrated in Fig. 2a and 1b, the molecules of the CMM surfaces 14 may modify the movement of the cells 4. The modified movements may be expressed as cells 4 taking a characteristic type of path if they interact with the CMM surfaces 14 of a zone 12.

In Fig. 2a, a biological cell 4' of the first category takes a more undulating path in the zone 12 compared to the path of the biological cell 4" of the second category.

In Fig. 2b, a biological cell 4' of the first category takes a more undulating path in the first zone 12' than in the second zone 12". Analogously, a biological cell 4" of the second category takes a more undulating path in the second zone 12" than in the first zone 12'.

The molecules may bind and release a cell 4 belonging to the associated cell category of the zone 12. For example, an anti-CD4 antibody may bind to a cell 4 having a cell membrane comprising CD4 antigens, and subsequently release the cell. Thus, the movements of a biological cell 4' of the first category moving through the first zone 12' may change in a characteristic way that makes it possible to identify the cell as belonging to the first category. The movements of a biological cell 4" of the second category moving through the second zone 12" may change analogously. When a biological cell belongs to a category associated with a zone the biological cell may, in that zone, exhibit e.g. a bind and release event, a halt of motion, a change of speed, a change of direction, or a change of path. For example, a biological cell that is observed to stick for a short time to a pillar in the first zone 12' may be regarded as a cell 4' of the first category. Alternatively, a biological cell that is observed to stick for a short time to a threshold number, e.g. 10, of pillars in the first zone 12' may be regarded as a cell 4' of the first category. In another example, biological cells that exhibit bind-roll-release type of movements in the first zone 12' may be regarded as a cell 4' of the first category. Fig. 2a and 1b illustrates a cell 4' of the first category which, in the first zone 12', follows the curvature of the pillars 17 to an extent that indicates that the cell does not merely follow the flow 2 of liquid.

A modified movement may be detected at a specific location of the zone 12, e.g. the biological cell 4 following the CMM surface 14 of a single pillar 17 of the zone 12 in a characteristic way. Alternatively, a modified movement may be detected over the entire zone 12 or over an area of the zone 12, e.g. the cell 4 taking an undulating path when interacting with several obstacles 16 of the zone 12.

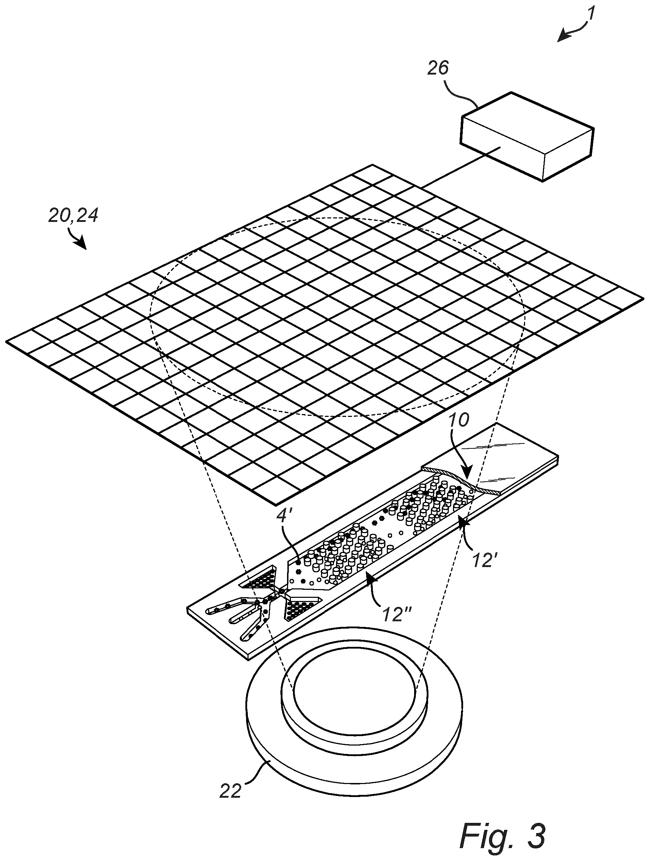

Fig. 3 illustrates a device 1 comprising a detector 20, being an image sensor 24 and a light source 22. The illustrated light source 22 illuminates the zones 12 of the flow channel 10. The flow channel 10 of the device 1 in Fig. 3 is the flow channel 10 depicted in Fig. 2b.

The illustrated light source 22 is configured to illuminate the cells 4 as they pass through the flow channel 10. The light source 22 may herein be a coherent light source 22, e.g. a laser, or a partially coherent light source 22, e.g. a light emitting diode or a light emitting diode with a pin hole or aperture. As the cells 4 are illuminated an interference pattern is formed on the image sensor 24. The image sensor 24 may comprise a plurality of photo-sensitive elements configured to detect incident light. The image sensor 24 may herein be a CCD or CMOS camera. The image sensor 24 may acquire a time-sequence of image frames of the changing interference pattern as cells 4 pass the image sensor 24. In the figure the light source 22 and the image sensor 24 are arranged on opposite sides of the flow channel 10. The flow channel 10 is herein at least partially transparent such that the light may travel through the flow channel 10.

The detector 20 is configured to detect information representing the biological cells 4, carried by the flow of liquid 2, passing the at least one zone 12.

The at least one detector 20 may be configured to detect information of at least one property of the biological cells 4. The property may be the size, shape or solidity of the biological cells 4.

The at least one detector 20 may be an optical sensor or the device 1 may further comprise an optical sensor. The at least one detector 20 may be an electrical sensor or the device 1 may further comprise an electrical sensor.

The device 1 further comprises a processor 26 which is configured to receive the information detected by the at least one detector 20.

The processor 26 may e.g. perform a holographic reconstruction of two interference patterns detected by the detector 20 in the time-sequence of interference patterns. Said two reconstructions may depict e.g. a biological cell 4 in contact with a CMM surface 14. If both reconstructed images show the biological cell 4 at the same location it may be deduced that the biological cell is stuck to the CMM surface and therefore has an affinity to the CMM surface 14. If both reconstructed images show the biological cell 4 in contact with the CMM surface but at different locations, it may be deduced that the cell has stuck to the CMM surface 14 and thereafter rolled along the CMM surface 14. In either case, the biological cell 4 may be detected as exhibiting a modified movement.

The processor 26 may not necessarily perform a full holographic reconstruction of two interference patterns in the time-sequence of interference patterns. The cell 4 may be detected as exhibiting a modified movement based on two, or more, interference patterns in the time-sequence of interference patterns or partial holographic reconstructions of the two, or more, interference patterns.

The processor 26may be configured to detect one or a plurality of modified movement(s) of a biological cell 4 in a zone 12, e.g. one bind and release event, a halt of motion, a change of speed, a change of direction, or a change of path, from the information received from the at least one detector 20, and thereby identify the biological cell 4 as belonging to the cell category associated with the zone 12. Alternatively, the processor 26 may be configured to process the information from the at least one detector 20 and identify a threshold number of modified movements of a biological cell 4 in a zone 12, e.g. the biological cell 4 displaying at least 2, at least 5, or at least 10 bind and release events within the zone, and thereby identify the biological cell 4 as belonging to the cell category associated with the zone 12. The processor 26 may be configured to identify modified movements of two or more biological cells 4 simultaneously, as they move across a CMM surface of the flow channel.

The device 1 may be configured to detect modified movements of biological cells carried by the flow of liquid in a manner described in WO2022/241138 A1, which is hereby incorporated by reference.

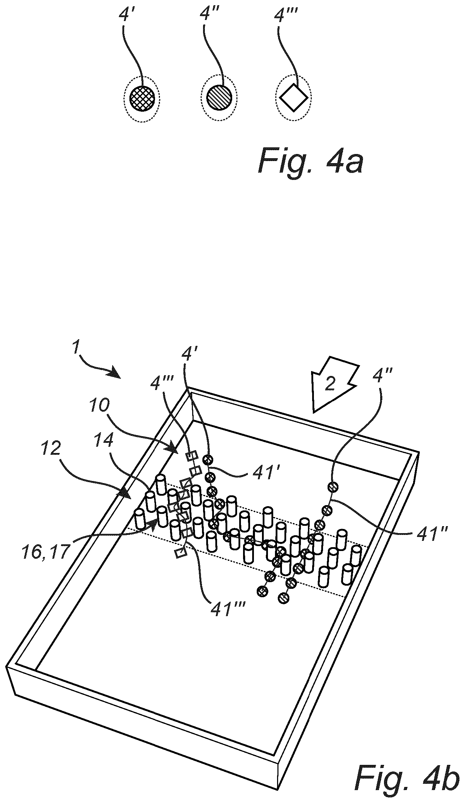

The processor 26 is configured to process the information received from the at least one detector 20. The processing of the information will now be discussed in relation to Fig. 4a to g.

The processor 26 may be a central processing unit (CPU) or a graphic processing unit (GPU), which may execute the instructions of one or more computer programs in order to process the information received from the at least one detector 20.

The processor 26 may alternatively be implemented as firmware arranged e.g. in an embedded system, or as a specifically designed processing unit, such as an Application-Specific Integrated Circuit (ASIC) or a Field-Programmable Gate Array (FPGA), which may be configured to implement functionality for processing the information received from the at least one detector.

The processing of the information comprises identifying candidate biological cells 4, as illustrated in Fig 4a where the candidate biological cells 4', 4" and 4‴ are identified. These may be identified in information at a single point in time from the at least one detector 20. The candidate biological cells 4 may be biological cells 4 belonging to the cell category of the zone 12. For instance, the candidate biological cell 4' of Fig. 2b belongs to the associated cell category with the first zone 12', while the candidate biological cell 4" belongs to the associated cell category of the second zone 12". However, one candidate biological cell 4', 4" may belong to more than one cell category. Thus, one candidate biological cell 4', 4" may belong to both the associated cell category with the first zone 12' and the second zone 12". The candidate biological cells 4 may be a biological cell 4 not belonging to the cell category of the zone 12. For instance, the candidate biological cell 4' does not associate with the cell category of zone 12". The candidate biological cell 4 may however also be non-cell 4‴. The non-cell 4‴ could be a debris in the flow of liquid, a dead or injured cell, an artefact in the CMM surface of the zone or an imaging artefact interpreted by the detector as a cell.

Further, the processor 26 is configured to identify tracks 41', 41", 41‴ from each of the candidate biological cells 4', 4", 4'". This is illustrated in Fig. 4b. The track 41' corresponds to a time sequence of identified locations of the identified candidate biological cell 4' in sequential time points of the information from the at least one detector 20. The track 41" corresponds to a sequence of identified locations of the identified candidate biological cell 4". The track 41‴ corresponds to a sequence of identified locations of the identified candidate biological cell 4‴. Thus, each identified candidate biological cell 4', 4", 4‴ each has its own track 41', 41", 41‴.

The tracking monitors the position of the detected candidate biological cells 4', 4", 4‴ in the flow channel 10 over a time-sequence during which the candidate biological cell 4', 4", 4‴ passes the zone 12. Thus, the movements of the each of the candidate biological cell 4', 4", 4‴ passing the zone 12 is represented by the tracks 41', 41", 41‴.

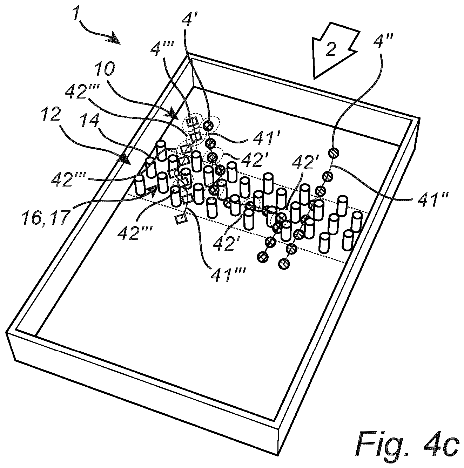

Further, the processor 26 is configured to identifying candidate modified movements as events 42', 42", 42‴ in each track of the candidate biological cells 4', 4", 4'". This is illustrated in Fig 4c. For simplicity, features not relevant for the illustration of events 42', 42", 42‴ are removed from Fig 4c. In Fig 4c, three events 42', 42‴ are identified for the candidate biological cells 4' and 4'", while the cell 4" had no events 42".

For each event 42', 42", 42'", the processor 26 is configured to determine event related information associated with the event 42', 42", 42'". The event related information comprises at least one of a sensed property of the candidate biological cells 4 and/or a property defining a relation between events 42', 42", 42‴ of at least two tracks 41', 41", 41‴ from candidate biological cells 4.

The processor 26 may be configured to process information detected by an optical sensor for determining an optical property of each of the candidate biological cells 4. The optical sensor may be an array of optical sensors. The optical property of the candidate biological cells 4 comprises the size, shape, orientation, granularity, homogeneity, smoothness, patchiness, transparency, scattering, brightness or solidity of the candidate biological cells 4.

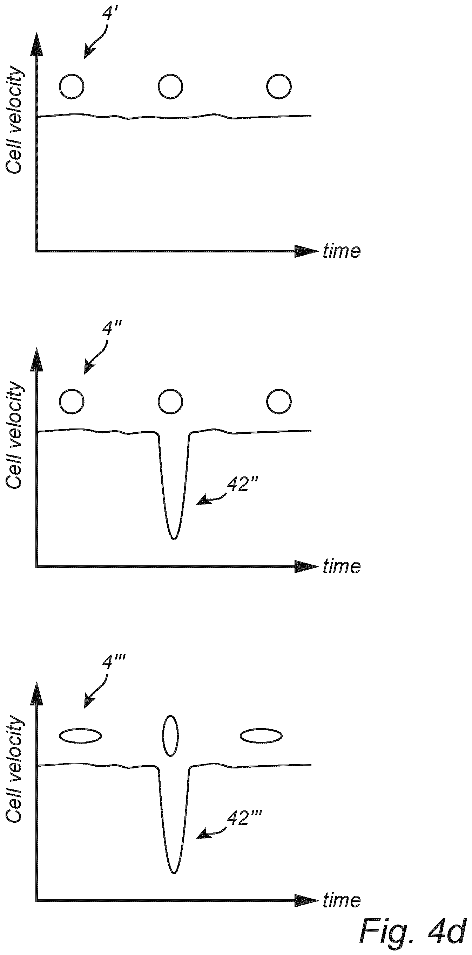

The optical property may for instance further affect the movement, such as the velocity, of the candidate biological cell 4. This is illustrated in Fig. 4d, illustrating the velocity of the candidate biological cell 4', 4" and 4‴ over time. As can be seen, the candidate biological cell 4' has the same shape and velocity over the entire time, giving rise to no event. The candidate biological cell 4" also has the same shape over the time period, however the velocity is decreased during a short time giving rise to an event 42". This event 42" is very likely connected to an interaction between the candidate biological cell 4" and the CMM surface 14. Thus, this event 42" may comprise relevant information for the characterization of the candidate biological cell 4". The candidate biological cell 4‴ however, has a change in morphology at the same time-point as the change in velocity. This may indicate that the event 42‴ identified at that time point is not connected to an interaction between the candidate biological cell 4‴ and the CMM surface, but only to a change in shape or orientation of the candidate biological cell 4‴. This may be decided based on this event related information, or more event related data of the candidate biological cell 4‴ may be acquired over time and together the data may indicate if the candidate biological cell 4‴ is a biological cell of interest or not.

The processor 26 may be configured to process information detected by an electrical sensor for determining an electrical property of each of the candidate biological cells 4. The electrical sensor may be an array of electrical sensors. The electrical property of the candidate biological cells 4 comprises the electrical impedance, at one or more frequencies, of the candidate biological cells 4, wherein the electrical impedance of the candidate biological cells 4 is used to determine the size, membrane capacitance or cytoplasm conductivity of the candidate biological cells 4.

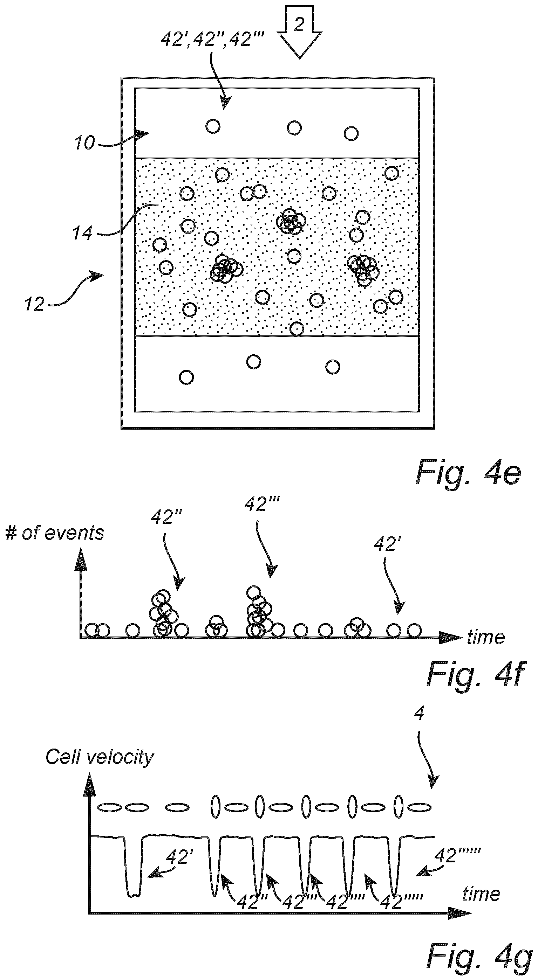

Determining event related information may for instance comprise determining a spatial property of the event 42', 42", 42'". This is illustrated in Fig. 4e, where several events 42', 42", 42'", here illustrated as open circles, happen at the same location, but at different time points, of the zone 12. This may indicate that there is a defect of the zone 12, a non-cell 4‴ (not visible in Fig. 4e) may be located there or a biological cell 4', 4" (not visible in Fig. 4e), may be stuck at the location. This may affect the following cells such that their behavior while or after passing the location is not correct. Thus, the events 42', 42", 42‴ associated with the location may induce one or more errors in the tracks 41', 41", 41‴.

Determining event related information may for instance comprise determining a temporal property of the event 42', 42", 42'". This is illustrated in Fig. 4f where several events 42", 42‴ happen at the same time, but at different locations in the flow channel 10. This may indicate that the flow of liquid 2 is irregular. Comparing the event 42' with the events 42" and 42‴ may reveal that the events 42", 42‴ may comprise errors such that candidate biological cells 4 have made modified movements not induced by the CMM surface 14 of the zone 12 but rather for instance the irregular flow of liquid 2.

Determining a temporal property of an event 42', 42", 42‴ may further comprise determining a repetition of the temporal property of the event 42', 42", 42'". This is illustrated in Fig. 4g, where the temporal property of the event 42' differs from the very regular temporal component of, and interval between the events 42", 42'", 42"", 42'"", 42""". This may indicate that the events 42", 42'", 42"", 42'"", 42""" are not caused by an interaction between the candidate biological cell 4 and the CMM surface 14. This may reveal that the events 42", 42'", 42"", 42'"", 42""" are caused by a change in orientation of the candidate biological cell 4 which was not detected by the detector, but which has altered the velocity. A combination of the event related information in connection with the event 42', and the event related information in connection with the events 42", 42'", 42"", 42'"", 42""", the candidate biological cell 4 may be characterized.

Determining a relation between events 42', 42", 42‴ of at least two tracks 41', 41", 41‴ from candidate biological cells 4 comprises comparing spatial and/or temporal properties of events 42', 42", 42‴ of the at least two tracks 41', 41", 41‴. Determining event related information may comprise determining whether a multitude, such as at least four, of events 42', 42", 42‴ in respective tracks 41', 41", 41‴ share a spatial and/or a temporal property. The multitude of events 42', 42", 42‴ may be 2, 10, 100 or 1000 depending on the experiment. This may include the examples as disclosed above, where the determination of the spatial or temporal property is made by comparing two or more tracks 41', 41", 41‴. Then, regularity between events 42', 42", 42‴ in space or time may be found.

This may further comprise determining a property based on the candidate biological cells 4 for at least two tracks 41', 41", 41‴, at the same time, are within a spatial distance to each other smaller than a threshold. It may for instance comprise that the property defines a relation between events 42', 42", 42‴ of at least two tracks 41', 41", 41‴ from candidate biological cells 4 indicating encountering of at least two candidate biological cells 4. At least two candidate biological cells 4 may interact with each other. The interaction between the at least two candidate biological cells 4 may induce errors in the event related information from the event 42', 42", 42'". The candidate biological cells 4 involved in the encounter may for instance physically overlap during a time, such that the event related information cannot decide the properties of each candidate biological cells 4. The candidate biological cells 4 may interact such that the track 41', 41", 41‴ may shift between the candidate biological cells 4. This may happen if the candidate biological cells 4 changes place with each other such that the processor 26 believes that the associated candidate biological cell 4 is still in one place while it actually is a second candidate biological cell taking its place. Then, the information of the tracking 41', 41", 41‴ is acquired from two candidate biological cells 4 if the tracking 41', 41", 41‴ is not corrected. The candidate biological cell 4', 4", 4‴ may push each other such that a non-specific modified movement is detected.

The property defining a relation between events 42', 42", 42‴ indicates that an association between a particular track 41', 41", 41‴ and a particular candidate biological cell 4', 4", 4‴ is lost or generated inside the zone 12. A candidate biological cell 4 may from time-to-time fade, for instance while moving and by stretching. This may be interpreted as if the candidate biological cell 4 disappears and the tracking 41', 41", 41‴ may again move to the closest candidate biological cell 4, leading to a possible cascade of tracking errors when each tracking 41', 41", 41‴ is forced to move to another candidate biological cell 4. When the fading candidate biological cell 4 is again reappearing, the tracking 41', 41", 41" may return to it. This of course induces errors between the fading and appearing of the candidate biological cell 4.

The processor 26 may identify the candidate biological cell 4 as a biological cell of interest 4', 4", a biological cell not of interest 4', 4" or a non-cell 4'", based on the event related information comprising the property of the candidate biological cells 4.

The processor 26 may identify too many and/or too regular events 42', 42", 42'", such that it is a high probability that the event 42', 42", 42‴ is not necessarily caused by the CMM surface.

The event related information is further used by the processor 26 to identify candidate non-specific modified movements among the candidate modified movements for each track 41', 41", 41‴ based on the event related information indicating that the event 42', 42", 42‴ is not necessarily caused by the CMM surface.

As is exemplified above, there are several events 42', 42", 42‴ that may appear where the event related information may reveal that there are errors in the track 41', 41", 41‴. For a further analysis or processing of the biological cells 4 it is of high importance that the tracking 41', 41", 41‴ is correct. Therefor, the processor uses the event related information to determine what events 42', 42", 42‴ are possibly caused by the CMM surface and what events 42', 42", 42‴ are necessarily not caused by the CMM surface.

Thus, candidate non-specific modified movements may be movements of candidate biological cells 4 which moves in a certain way because it was affected by anything other than the CMM surface. As is exemplified above, the candidate biological cell 4 may be affected by a stuck non-cell 4‴ or by an artefact of the zone 12.

The candidate non-specific modified movement may as well be a tracking error, such that the tracking 41', 41", 41‴ of one candidate biological cell 4 moves to another candidate biological cell 4. Thus, the candidate biological cell 4 may have a specific modified movement, but the tracking 41', 41", 41‴ may for a short while be attached to the wrong candidate biological cell 4.

The processor 26 removes the candidate non-specific modified movements from the candidate modified movements for each track, 41', 41", 41‴ whereby remaining candidate modified movements for each track 41', 41", 41‴ has an increased portion of specific modified movements.

By this, the remaining portion of specific modified movements are a more accurate data of the biological cells 4 belonging to the associated biological cell category of the zone 12. Thus, the information in the data is more accurate for further processing.

The processor 26 may be configured to determine that an entire track 41', 41", 41‴ is to be disregarded based on the determined event related information of one or more events of the track 41', 41", 41‴. Thus, if the track 41', 41", 41‴ includes such errors that it may not be of relevance to analyze or if the candidate biological cell 4 is identified as a non-cell 4‴ the entire track 41', 41", 41‴ may be removed.

For instance, a removal of the entire track may appear if the processor 26 cannot decide on the category of the candidate biological cell 4', 4", 4'". Thus, if the processor 26 cannot decide of the candidate biological cell 4', 4", 4" is associated of not to the zone 12', 12". The processor 26 may be configured to analyze each of the remaining part of the tracks 41', 41", 41‴ based on remaining candidate modified movements of the track 41', 41", 41‴ to determine whether the track 41', 41", 41‴ represents movements of the biological cell 4 belonging to the associated biological cell category of the zone 12.

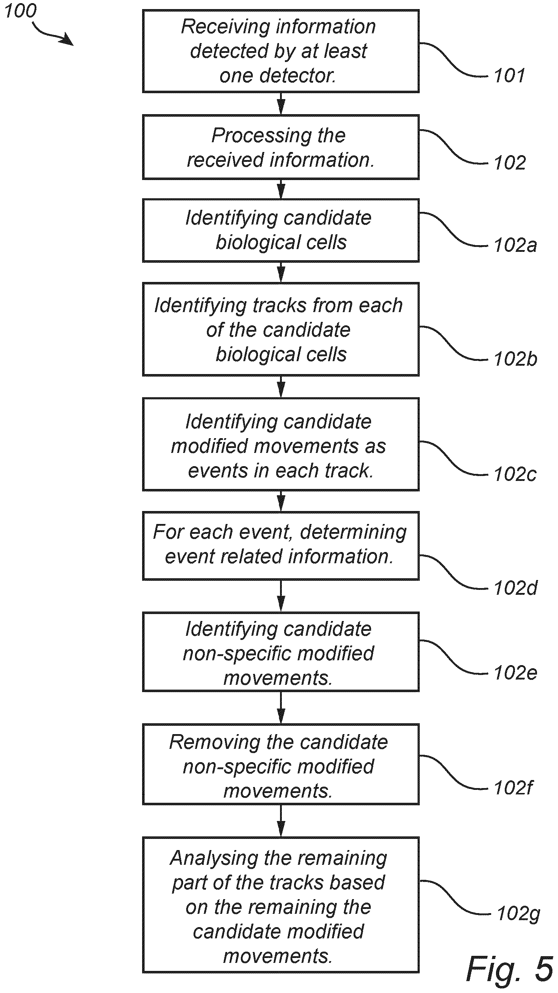

Fig. 5 illustrates a flow chart of a method 100 for analyzing biological cells 4, 4', 4", 4‴. The method 100 comprises receiving 101 information detected by at least one detector 20 representing biological cells 4, 4', 4", 4'", carried by a flow of liquid 2, to be analyzed passing at least one zone 12, 12', 12" in a flow channel 10.

Each zone 12, 12', 12" of the flow channel 10 is associated with a cell category. Each zone 12, 12', 12" of the flow channel 10 comprises at least one surface coated with molecules having an affinity to the cell category associated with the zone 12. The at least one surface of the zone 12 being configured to modify, by the molecules coating the at least one surface, a movement of a biological cell 4, 4', 4", 4‴ belonging to the associated cell category of the zone 12 as the biological cell 4, 4', 4", 4‴ carried by the flow of liquid 2 passes the zone 12. The at least one surface forms at least one biological cell movement modifying (CMM) surface 14. The modified movement of the biological cell 4, 4', 4", 4‴ caused by the CMM surface 14 forms a specific modified movement.

The method 100 further comprises processing 102 the received information.

The processing 102 of the information comprises identifying 102a candidate biological cells 4, 4', 4", 4‴ in the flow channel 10.

The processing 102 of the information further comprises identifying 102b tracks 41', 41", 41‴ from each of the candidate biological cells 4, 4', 4", 4'". Thus, each of the identified 102a candidate biological cells 4, 4', 4", 4‴ receives a track 41', 41", 41‴ which follows the movements of the candidate biological cell 4, 4', 4", 4'". The track 41', 41", 41‴ acquires information on movements of the candidate biological cells 4, 4', 4", 4‴ as well as other properties of the candidate biological cells 4, 4', 4", 4'".

The processing 102 of the information further comprises identifying 102c candidate modified movements as events 42', 42", 42‴ in each track of the candidate biological cells 4, 4', 4", 4'".

For each event 42', 42", 42'", the processing 102 comprises, determining 102d event related information associated with the event 42', 42", 42'". The event related information comprises at least one of a property of the candidate biological cells 4, 4', 4", 4‴ and/or a property defining a relation between events 42', 42", 42‴ of at least two tracks 41', 41", 41‴ from candidate biological cells 4, 4', 4", 4‴. Thus, the track 41', 41", 41‴ provides with information of a property of the candidate biological cell 4, 4', 4", 4‴ or the relationship between at least two candidate biological cells 4, 4', 4", 4‴. In other words, the tracking may give morphological information on the candidate biological cell 4, 4', 4", 4‴ or spatial or temporal information on its movement, alone or in relation to other candidate biological cells 4, 4', 4", 4‴.

The processing 102 of the information further comprises identifying 102e candidate non-specific modified movements among the candidate modified movements for each track 41', 41", 41‴ based on the event related information indicating that the event 42', 42", 42‴ is not necessarily caused by the CMM surface 14.

By using the event related information, errors in the tracking 41', 41", 41‴ may be identified. The errors may otherwise give wrong interpretation of the data.

The processing 102 of the information further comprises removing 102f the candidate non-specific modified movements from the candidate modified movements for each track 41', 41", 41‴. The remaining candidate modified movements for each track 41', 41", 41‴ then has an increased portion of specific modified movements. Thus, errors from the tracking 41', 41", 41‴ may be removed, such that the data is more correct.

The processing 102 of the information further comprises analyzing 102g the remaining part of the tracks 41', 41", 41‴ based on remaining candidate modified movements of the track 41', 41", 41‴ to determine whether the track 41', 41", 41‴ represents movements of the biological cell 4, 4', 4", 4‴ belonging to the associated biological cell category of the zone.

The processing 102 may further comprise a repetition of 102c, 102d, 102e, 102f and/or 102g to increase the proportion of tracks 41', 41", 41‴ representing movements of the biological cell 4, 4', 4", 4‴ belonging to the associated biological cell category of the zone.

By this, the method 100 provides with an analysis which allows for high throughput and low hands on from the user but still with a high precision of the analysis. Errors occurring in the tracking 41', 41", 41‴ are handled in the processing 102 of the information and the final analysis 102g of the data allows for a more precise analysis with reduced errors.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A device (1) for analyzing biological cells (4, 4', 4", 4‴) comprising:
a flow channel (10) configured to pass a flow of liquid (2), wherein:
the flow of liquid (2) carries the biological cells (4, 4', 4", 4‴) to be analyzed,
the flow channel (10) comprises at least one zone (12, 12', 12"),
each zone (12, 12', 12") of the flow channel (10) is associated with a cell category, and
each zone (12, 12', 12") of the flow channel (10) comprises at least one surface coated with molecules having an affinity and specificity to the cell category associated with the zone (12, 12', 12"), the at least one surface of the zone (12, 12', 12") being configured to modify, by the molecules coating the at least one surface, a movement of a biological cell (4, 4', 4", 4‴) belonging to an associated biological cell category of the zone (12, 12', 12") as the biological cell (4, 4', 4", 4‴), carried by the flow of liquid (2), passes the zone (12, 12', 12"), whereby the at least one surface forms at least one cell movement modifying (CMM) surface (14), and whereby the modified movement of the biological cell (4, 4', 4", 4‴) caused by the CMM surface (14) forms a specific modified movement;
at least one detector (20) configured to detect information representing the biological cells (4, 4', 4", 4‴), carried by the flow of liquid (2), passing the at least one zone (12, 12', 12");
a processor (26) configured to receive the information detected by the at least one detector (20), and to process the information, wherein processing of the information comprises:
identifying candidate biological cells (4, 4', 4", 4‴);
identifying tracks (41', 41", 41‴) from each of the candidate biological cells (4, 4', 4", 4‴);
identifying candidate modified movements as events (42', 42", 42‴, , 42ʺʺ, 42‴ʺ, 42‴‴) in each track of the candidate biological cells (4, 4', 4ʺ. 4‴);
for each event (42', 42", 42‴, 42ʺʺ, 42‴ʺ, 42‴‴), determining event related information associated with the event (42', 42", 42‴, 42ʺʺ, 42'"", 42"""), wherein the event related information comprises at least one of a sensed property of the candidate biological cells (4, 4', 4", 4‴) and/or a property defining a relation between events (42', 42", 42‴, 42ʺʺ, 42‴ʺ, 42‴‴) of at least two tracks (41', 41", 41‴) from candidate biological cells (4, 4', 4", 4‴);
identifying candidate non-specific modified movements among the candidate modified movements for each track (41', 41", 41‴) based on the event related information indicating that the event (41', 41", 41‴) is not necessarily caused by the CMM surface (14); and
removing the candidate non-specific modified movements from the candidate modified movements for each track (41', 41", 41‴), whereby remaining candidate modified movements for each track (41', 41", 41‴) has an increased portion of specific modified movements.

2. A device (1) for analyzing biological cells (4, 4', 4", 4‴) according to claim 1, wherein the at least one detector (20) is an optical sensor or wherein the device (1) further comprises an optical sensor, and wherein the processor (26) is configured to process information detected by the optical sensor for determining an optical property of each of the candidate biological cells (4, 4', 4", 4‴), wherein the property of the candidate biological cells (4, 4', 4", 4‴) comprises an optical property of the candidate biological cell (4, 4', 4", 4‴), wherein the optical property of the candidate biological cell (4, 4', 4", 4‴) is event related information used to identify candidate non-specific modified movements.

3. A device (1) for analyzing biological cells (4, 4', 4", 4‴) according to claim 2, wherein the optical property of the candidate biological cells (4, 4', 4", 4‴) comprises the size, shape, orientation, granularity, homogeneity, smoothness, patchiness, transparency, scattering, brightness or solidity of the candidate biological cells (4, 4', 4", 4‴).

4. A device (1) for analyzing biological cells (4, 4', 4", 4‴) according to any one of the preceding claims, wherein the at least one detector (20) is an electrical sensor or wherein the device (1) further comprises an electrical sensor, and wherein the processor (26) is configured to process information detected by the electrical sensor for determining an electrical property of each of the candidate biological cells (4, 4', 4", 4‴), wherein the property of the candidate biological cells (4, 4', 4", 4‴) comprises an electrical property of the candidate biological cell (4, 4', 4", 4‴).

5. A device (1) for analyzing biological cells (4, 4', 4", 4‴) according to claim 4, wherein the electrical property of the candidate biological cells (4, 4', 4", 4‴) comprises the electrical impedance at one or more frequency, of the candidate biological cells (4, 4', 4", 4‴), wherein the electrical impedance of the candidate biological cells (4, 4', 4", 4‴) is used to determine the size, membrane capacitance and/or cytoplasm conductivity of the candidate biological cells (4, 4', 4", 4‴).

6. A device (1) for analyzing biological cells (4, 4', 4", 4‴) according to any one of claims 1-5, wherein the candidate biological cell (4, 4', 4", 4‴) is identified as a biological cell of interest (4', 4"), a biological cell not of interest (4', 4") or a non-cell (4‴), based on the event related information comprising the property of the candidate biological cells (4, 4', 4", 4‴).

7. A device (1) for analyzing biological cells (4, 4', 4", 4‴) according to any one of the preceding claims, wherein determining event related information comprises determining a spatial and/or temporal property of the event (42', 42", 42‴), and wherein determining a relation between events (42', 42", 42'", 42"", 42'"", 42""") of at least two tracks (41', 41", 41‴) from candidate biological cells (4, 4', 4", 4‴) comprises comparing spatial and/or temporal properties of events (42', 42", 42‴, 42ʺʺ, 42‴ʺ, 42‴‴) of the at least two tracks (41', 41", 41‴).

8. A device (1) for analyzing biological cells (4, 4', 4", 4‴) according to claim 7, wherein determining event related information comprises determining a property based on the candidate biological cells (4, 4', 4", 4‴) for at least two tracks (41', 41", 41‴) being within a spatial distance to each other smaller than a threshold.

9. A device (1) for analyzing biological cells (4, 4', 4", 4‴) according to claim 8, wherein the property defining a relation between events (42', 42", 42'", 42ʺʺ, 42‴ʺ, 42‴‴) of at least two tracks (41', 41", 41‴) from candidate biological cells (4, 4', 4", 4‴) indicates encountering of at least two candidate biological cells (4, 4', 4", 4‴).

10. A device (1) for analyzing biological cells (4, 4', 4", 4‴) according to any one of the preceding claims, wherein the property defining a relation between events (42', 42", 42‴, 42ʺʺ, 42‴ʺ, 42‴‴) indicates that an association between a particular track (41', 41", 41‴) and a particular candidate biological cell (4, 4', 4", 4‴) is lost or generated inside the zone (12, 12', 12").

11. A device (1) for analyzing biological cells (4, 4', 4", 4‴) according to any one of claims 7-10, wherein determining event related information comprises determining whether a multitude, such as at least four, of events (42', 42", 42'", 42"", 42'"", 42""") in respective tracks (41', 41", 41‴) share a spatial and/or a temporal property.

12. A device (1) for analyzing biological cells (4, 4', 4", 4‴) according to claim 1, wherein the processor (26) is configured to determine that the entirety of a track (41', 41", 41‴) before and/or after an event (42', 42", 42'", 42"", 42‴ʺ, 42""") is to be disregarded based on the determined event related information of the event (42', 42", 42'", 42"", 42‴ʺ, 42‴‴) of the track (41', 41", 41‴).

13. A device (1) for analyzing biological cells (4, 4', 4", 4‴) according to any one of claims 1-12, wherein the device (1) further comprises:
a light source (22) configured to illuminate a biological cell (4, 4', 4", 4‴) in the flow channel (10) such that an interference pattern is formed by interference between light being scattered by the illuminated biological cell (4, 4', 4", 4‴) and non-scattered light from the light source (22); and
wherein the detector (20) comprises an image sensor (24) configured to detect an image series that represents a time-sequence of interference patterns of the illuminated biological cell (4, 4', 4", 4‴).

14. A method (100) for analyzing biological cells (4, 4', 4", 4‴), the method (100) comprising:
receiving (101) information detected by at least one detector (20) representing biological cells (4, 4', 4", 4‴), carried by a flow of liquid (2), to be analyzed passing at least one zone (12, 12', 12") in a flow channel (10), wherein
each zone (12, 12', 12") of the flow channel (10) is associated with a cell category,
each zone (12, 12', 12") of the flow channel (10) comprises at least one surface coated with molecules having an affinity to the cell category associated with the zone (12), the at least one surface of the zone (12) being configured to modify, by the molecules coating the at least one surface, a movement of a biological cell (4, 4', 4", 4‴) belonging to the associated cell category of the zone (12) as the biological cell (4, 4', 4", 4‴) carried by the flow of liquid (2) passes the zone (12), whereby the at least one surface forms at least one biological cell movement modifying (CMM) surface (14), and whereby the modified movement of the biological cell (4, 4', 4", 4‴) caused by the CMM surface (14) forms a specific modified movement,
processing (102) the received information, wherein said processing (102) of the information comprises:
identifying (102a) candidate biological cells (4, 4', 4", 4‴) in the flow channel (10);
identifying (102b) tracks (41', 41", 41‴) from each of the candidate biological cells (4, 4', 4", 4‴);
identifying (102c) candidate modified movements as events (42', 42", 42‴) in each track of the candidate biological cells (4, 4', 4", 4‴);
for each event (42', 42", 42‴, 42"", 42'"", 42‴‴), determining (102d) event related information associated with the event (42', 42", 42‴), wherein the event related information comprises at least one of a sensed property of the candidate biological cells (4, 4', 4", 4‴) and/or a property defining a relation between events (42', 42", 42‴, 42ʺʺ, 42‴ʺ, 42‴‴) of at least two tracks (41', 41", 41‴) from candidate biological cells (4, 4', 4", 4‴);
identifying (102e) candidate non-specific modified movements among the candidate modified movements for each track (41', 41", 41‴) based on the event related information indicating that the event (42', 42", 42‴, 42"", 42'"", 42""") is not necessarily caused by the CMM surface (14); and
removing (102f) the candidate non-specific modified movements from the candidate modified movements for each track (41', 41", 41‴), whereby remaining candidate modified movements for each track (41', 41", 41‴) has an increased portion of specific modified movements.

15. A computer program (300) product comprising computer-readable instructions such that when executed on a processor, the computer program product will cause the processor to perform the method according to claim 14.
